# FASCICULE DE BREVET EUROPEEN

(11) **EP 3 824 817 B1**
(45) Date de publication et mention de la délivrance du brevet: **01.01.2025**
(21) Numéro de dépôt: 19315138.8
(22) Date de dépôt: 22.11.2019
(51) Int. Cl.: A61B 8/08, A61B 1/00, A61B 8/12, A61B 8/00

(54) **CATHÉTER AVEC CAPTEURS INCORPORÉS D'IMAGERIE PAR ULTRASONS**
KATHETER MIT EINGEBAUTEN ULTRASCHALL BILDGEBUNGSSENSOREN
CATHETER WITH ULTRASOUND IMAGING SENSORS

(43) Date de publication de la demande: 26.05.2021
(73) Titulaire: Cairdac, 92160 Antony (FR)
(72) Inventeur: Regnier, Willy, 91160 Longjumeau (FR); Makdissi, Alaa, 75011 Paris (FR); Nguyen-Dinh, An, 37520 La Riche (FR); Ferin, Guillaume, 37038 Tours Cedex 1 (FR)
(74) Mandataire: Dupuis-Latour, Dominique

(56) Documents cités:
- WO-A1-2006/091597
- US-A1- 2014 180 126
- US-A1- 2015 196 309

## Description

### CONTEXTE DE L'INVENTION

### Domaine de l'invention

L'invention concerne un instrument d'exploration et d'intervention intracorporelles, placé à l'extrémité d'un cathéter introduit dans le corps d'un patient et portant à son extrémité distale un réseau de transducteurs pouvant être dirigés vers un site d'exploration ou d'intervention.

Les transducteurs peuvent notamment être des transducteurs à ultrasons. Par mise en oeuvre de techniques d'imagerie en elles-mêmes connues, le réseau de transducteurs permet alors d'obtenir une image échographique du site visé.

Ce type de transducteur n'est toutefois pas limitatif de l'invention, cette dernière n'étant pas dépendante, comme on le verra, de la technologie particulière de transducteur pour sa mise en oeuvre.

L'invention vise plus spécifiquement le cas où le cathéter est un cathéter creux, avec sur toute sa longueur une lumière axiale interne qui débouche côté distal face au site d'exploration ou d'intervention.

De manière en elle-même connue, un tel cathéter creux permet par exemple d'introduire un instrument dans la lumière et de manipuler celui-ci depuis l'extérieur du corps, ou encore de commander l'implantation d'un dispositif médical en un site précis de l'organisme. Dans ce dernier cas, le dispositif est initialement placé à l'extrémité distale du cathéter dans un "housing", c'est-à-dire un manchon de protection ; ensuite, une fois le site d'implantation reconnu et atteint, le dispositif est repoussé hors du housing par un cathéter interne ou autre moyen de commande disposé dans la lumière interne du cathéter principal.

### Description de la technique antérieure

Cette technique est par exemple utilisée - entre autres et de façon nullement limitative - pour l'implantation d'un dispositif de type capsule autonome implantable.

Un tel dispositif, ci-après désigné "capsule autonome", "capsule leadless" ou simplement "capsule" se présente sous forme d'une capsule implantée dans une cavité cardiaque (ventricule, oreillette ou même cavité cardiaque gauche artérielle). La capsule est autonome, c'est-à-dire qu'elle est dépourvue de toute liaison physique à un dispositif principal implanté (tel qu'un boîtier de générateur d'impulsions de stimulation) ou non implanté (périphérique externe tel que programmateur ou dispositif de monitoring pour le suivi à distance du patient). Pour cette raison, ce type de dispositif est dénommé "leadless", pour le distinguer des électrodes ou des capteurs disposés à l'extrémité distale d'une sonde (Iead) conventionnelle parcourue sur toute sa longueur par un ou plusieurs conducteurs reliant par voie galvanique l'électrode ou le capteur à un générateur connecté à une extrémité opposée, proximale, de la sonde.

Les US 2009/0171408 A1 (Solem), US 2017/0151429 A1 (Regnier) et WO 2018/122244 A1 (Regnier) décrivent divers exemples de telles capsules leadless intracardiaques.

L'implantation de telles capsules endocavitaires implique des contraintes importantes, notamment du fait de la voie d'approche qui nécessite de passer par le réseau périphérique veineux. En effet, du fait des dimensions relativement importantes des capsules leadless actuelles qui ont un diamètre typique d'environ 4 à 7 mm pour une longueur de 15 à 40 mm, avec un objet d'une telle taille il n'existe pas de procédure par voie haute, c'est-à-dire via la veine sous-clavière, pour accéder à une cavité cardiaque, notamment pour atteindre le fond du ventricule droit. Il est de ce fait nécessaire d'utiliser un abord différent, en partant d'une ponction fémorale puis en remontant la veine cave inférieure jusqu'au coeur.

La problématique est la même si l'on souhaite procéder à l'explantation d'une capsule déjà en place, que ce soir pour l'extraire complètement ou pour la repositionner à un site voisin (dans la suite on fera simplement référence à l' "implantation" d'une capsule, étant entendu qu'il s'agit aussi bien de considérer l'opération inverse d'explantation).

Il existe à cet effet des cathéters téléorientables ("steerables"), qui sont des outils d'implantation bien connus dont l'extrémité distale est manoeuvrable depuis une poignée située à l'extrémité opposée, côté proximal, de manière à pouvoir effectuer sous amplificateur de brillance une telle manoeuvre de réorientation dans l'oreillette.

Un tel cathéter téléorientable et la manière d'opérer l'implantation sont décrits notamment dans le US 2014/0378991 (Ollivier). L'outil d'implantation divulgué par ce document comprend un housing cylindrique de protection prolongeant le cathéter téléorientable dans sa partie distale et contenant la capsule à implanter. Cette capsule est couplée à un sous-cathéter (ou "cathéter de délivrance") inséré dans la lumière interne centrale du cathéter principal (ou "cathéter-guide"), et elle est maintenue en position rétractée dans l'embout pendant toute la durée de la manoeuvre d'approche. La capsule et le cathéter de délivrance sont temporairement liés par un mécanisme débrayable simple permettant un vissage complet de la capsule dans la paroi cardiaque puis son largage final. La configuration télescopique du cathéter de délivrance permet de projeter la capsule hors de l'embout de protection et au-delà de celui-ci sur plusieurs centimètres, autorisant en toute circonstance une approche complète et précise de la capsule jusqu'au fond du ventricule.

L'invention n'est toutefois pas limitée à cette application particulière de l'implantation d'un capsule leadless, ni même à une opération d'implantation d'autres types de dispositifs médicaux implantables, quelle que soit leur destination fonctionnelle, cardiaque ou autre.

On verra en effet que l'invention peut être mise en oeuvre dans des contextes très variés, indépendamment de l'application spécifique considérée : diffusion *in situ* d'un agent pharmacologique actif, ablation de tissus, etc.

Le problème à résoudre tient au fait que, dans de nombreuses techniques d'intervention ou d'implantation, la présence d'une lumière interne de relativement grand diamètre (pour pouvoir y introduire le dispositif médical éventuellement pourvu de son manchon de protection, ou l'instrument à avancer jusqu'au site d'intervention) ne permet pas de placer les transducteurs du réseau à l'extrémité distale du cathéter, là où débouche cette lumière, de la même façon qu'avec les sondes d'imagerie conventionnelles. En effet, dans ces sondes conventionnelles, par exemple celles des dispositifs endoscopiques d'imagerie par ultrasons utilisés en coelioscopie, les transducteurs du réseau sont disposés sur un support plat en forme de disque s'étendant dans un plan radial. De fait, l'intervention nécessite alors l'introduction de deux cathéters, à savoir un cathéter pour recueillir une image échographique, et un (ou plusieurs) autre(s) cathéter(s) portant les instruments chirurgicaux.

Dans le cas d'un abord par un réseau veineux, il n'est pas envisageable d'introduire plusieurs cathéters côte à côte. Si l'on veut réunir dans un seul et même outil les deux fonctions - exploration par le réseau de transducteurs et intervention via la lumière axiale interne - il est alors nécessaire de recourir à un réseau bidimensionnel de transducteurs montés autour de la lumière centrale sur une face support tournée vers le site d'implantation ou d'intervention,.

Le US 2008/0221448 A1 (Khuri-Yakub et al.) décrit une telle structure comprenant une tête de cathéter creux portant en périphérie frontale une couronne de transducteurs à ultrasons destinés à recueillir après un traitement d'imagerie approprié une représentation du site corporel visé afin de guider et assister l'intervention chirurgicale, en l'espèce une ablation locale de tissus.

L'inconvénient de ce dispositif est son diamètre hors-tout très important, du fait notamment de la présence de la couronne de transducteurs en extrémité distale, dont le diamètre vient s'ajouter à celui du cathéter proprement dit.

Avec une telle configuration, si l'on voulait utiliser ce dispositif pour une intervention avec un abord par voie veineuse, soit le diamètre hors-tout excessif interdirait cette approche, soit la lumière interne serait de diamètre trop faible pour y introduire une capsule leadless à implanter, dont on a indiqué plus haut que le diamètre peut atteindre typiquement 4 à 7 mm, sans compter le diamètre supplémentaire du housing dans lequel cette capsule est logée. En tout état de cause, si l'on réduit trop la surface frontale occupée par le réseau de transducteurs, la résolution de l'image obtenue sera excessivement dégradée.

Une autre approche est décrite dans le WO 2017/144288 A1 (Koninklijke Philips NV), où les transducteurs du réseau ne sont pas placés sur une face frontale du cathéter, mais sur des supports allongés rétractables rayonnant à partir du centre du cathéter à son extrémité distale, ces supports étant déployés "à la manière des pétales d'une fleur" une fois atteint le voisinage du site d'intervention. Il est ainsi possible de disposer après déploiement d'un réseau de transducteurs à ultrasons relativement étendu, qui permet d'obtenir une image assez précise du site visé, par exemple une tumeur vers laquelle sont focalisés les transducteurs des différents "pétales" déployés. L'instrument d'ablation de la tumeur est introduit par une ouverture centrale du cathéter qui, en variante, peut également servir à l'amenée d'un fluide de refroidissement ou de gonflage d'un ballonnet.

La réalisation d'un tel système est cependant délicate et coûteuse, dans la mesure où il est nécessaire de concevoir un système mécanique articulé pour le déploiement des "pétales" par rapport au corps du cathéter. De plus, l'inclinaison des réseaux de conducteurs portés par les pétales déployés aura une incidence non négligeable sur la précision et la qualité de l'image échographique finalement obtenue au moyen de ces transducteurs.

Le US 2014/0180126 A1 décrit une autre type de sonde d'exploration, où un capteur d'imagerie est intégré à la paroi d'un tube externe, en forme de ballonnet gonflable, d'un cathéter intravasculaire de délivrance d'un stent. Cet agencement permet d'explorer la région d'implantation au moment du gonflement du ballonnet, de manière à pourvoir éventuellement réajuster le positionnement de ce dernier dans le vaisseau.

L'un des buts de l'invention est de proposer un instrument d'exploration et d'intervention intracorporelles du type précité, c'est-à-dire comportant un tube de cathéter creux et pourvu à son extrémité distale d'un réseau de transducteurs, notamment à ultrasons, qui pallie l'ensemble des difficultés évoquées ci-dessus.

Il s'agit en particulier de proposer un tel instrument :
dont la structure mécanique soit simple, donc facile et économique à industrialiser, permettant le cas échéant de proposer un produit à usage unique de coût raisonnable ;
dont le diamètre externe soit suffisamment réduit pour permettre un accès à un site d'intervention par exemple en faisant cheminer le tube de cathéter dans le réseau veineux du patient ;
qui laisse subsister une lumière interne centrale de diamètre important, compatible notamment avec l'introduction et la manoeuvre d'un instrument d'intervention opératoire, ou qui puisse loger une capsule leadless et ses moyens d'implantation ; et
dont le réseau de transducteurs, une fois opérationnel face au site à explorer, soit suffisamment étendu et déploie un nombre suffisamment élevé de transducteurs élémentaires, pour permettre un contrôle électronique de l'image du site.

### RÉSUMÉ DE L'INVENTION

L'invention propose à cet effet un instrument d'exploration et d'intervention intracorporelles selon la revendication 1.

Selon diverses caractéristiques subsidiaires avantageuses :
les transducteurs du réseau sont montés sur le module d'imagerie avec l'axe de leur diagramme de rayonnement orienté perpendiculairement à la face support ;
en position repliée les extrémités proximales respectives du manchon interne et du manchon externe sont mutuellement rapprochées, et le module d'imagerie s'étend substantiellement dans le prolongement du manchon interne, au moins une partie de la face du module d'imagerie portant les transducteurs du réseau étant orientée radialement vers l'intérieur du tube déformable ; et en position déployée les extrémités proximales respectives du manchon interne et du manchon externe sont mutuellement éloignées, la face du module d'imagerie portant les transducteurs du réseau s'étendant au moins en partie dans un plan radial où les transducteurs sont orientés suivant une direction axiale parallèle à l'axe central du tube déformable ;
en particulier et de façon avantageuse, en position déployée la face du module d'imagerie portant les transducteurs du réseau s'étend avec une partie des transducteurs orientés suivant une direction axiale parallèle à l'axe central du tube déformable, et une autre partie des transducteurs orientés suivant une direction inclinée intersectant l'axe central du tube déformable ;
le module d'imagerie comprend un circuit imprimé flexible portant et interconnectant les transducteurs du réseau, ce circuit imprimé flexible pouvant avantageusement être prolongé côté proximal par un ou plusieurs rubans s'étendant jusqu'à une extrémité proximale du tube déformable et au-delà, les rubans portant des pistes conductrices de liaison aux transducteurs du réseau ;
le tube déformable comprend un ensemble de lamelles flexibles parallèles s'étendant chacune dans un plan axial, chacune de ces lamelles étant formée d'une seule pièce en matériau élastique, notamment un métal ou un alliage métallique, et s'étendant de l'extrémité proximale du manchon interne jusqu'à l'extrémité proximale du manchon externe en étant repliée sur elle-même au niveau du module d'imagerie ;
le manchon interne et le manchon externe comportent chacun une bague à leur extrémité proximale respective, un déplacement des deux bagues en translation axiale relative réalisant une transition entre la position repliée et la position déployée ;
l'instrument comprend en outre un cathéter téléorientable prolongeant le tube déformable à son extrémité proximale et comprenant un tube mobile logé à l'intérieur d'un tube intermédiaire. Le tube mobile et le tube intermédiaire sont coaxiaux, s'étendent d'une extrémité proximale à une extrémité distale du cathéter et sont montés télescopiquement l'un dans l'autre avec possibilité de rotation mutuelle et de translation axiale mutuelle, et le manchon interne et le manchon externe sont reliés en partie proximale respectivement au tube mobile et au tube intermédiaire du cathéter ;
en particulier et de façon avantageuse, le manchon interne peut être relié de façon découplable au tube mobile du cathéter, notamment par l'intermédiaire d'un embout comportant un organe d'accouplement du tube mobile du cathéter à une capsule autonome implantable apte à être logée à l'intérieur de la lumière axiale du manchon interne ;
avantageusement, cet embout est découplable du manchon interne sous l'effet d'une rotation du tube mobile dans le tube intermédiaire du cathéter, l'embout restant couplé au manchon interne pendant une translation axiale du tube mobile dans le tube intermédiaire du cathéter ;
dans une autre forme de mise en oeuvre, le tube mobile du cathéter peut être relié à un instrument chirurgical apte à être logé à l'intérieur de la lumière axiale du manchon interne.

### DESCRIPTION SOMMAIRE DES DESSINS

La Figure 1 est une vue d'ensemble montrant un instrument selon l'invention, avec un tube de cathéter associé à son extrémité proximale à une poignée de manoeuvre utilisable par le praticien chargé de l'intervention.
La Figure 2 est une vue agrandie de l'extrémité distale du tube de cathéter de l'instrument selon l'invention, correspondant au détail repéré Il sur la Figure 1.
La Figure 3 est une vue schématique d'un circuit flexible portant le réseau de transducteurs de l'instrument de la Figure 2 ainsi que les conducteurs de liaison à ces transducteurs, ce circuit flexible étant représenté à plat, sous forme déroulée.
Les Figures 4a à 4d sont des vues perspectives partiellement en coupe de l'extrémité distale du tube de cathéter de la Figure 2, logeant dans son volume intérieur une capsule leadless à implanter, ces figures respectives illustrant les positions successives prises par l'ensemble lors de l'opération de déploiement du réseau de transducteurs et de début de translation de la capsule leadless pour implantation au site choisi.
Les Figures 5a à 5c sont des vues en coupe par un plan axial de l'extrémité distale du tube de cathéter et de la capsule leadless, montrant de façon plus précise le développement du circuit flexible portant les transducteurs et le positionnement frontal de ces derniers une fois le réseau complètement déployé.
La Figure 6 est une vue agrandie en perspective de l'embout portant l'organe d'accouplement à la capsule leadless et permettant de manoeuvrer cette dernière en translation et en rotation et déployer simultanément le réseau de transducteurs.
Les Figures 7a à 7c sont des vues isolées de l'extrémité distale du circuit flexible portant les transducteurs et illustrant le déploiement progressif du circuit flexible et du réseau de transducteurs, ces figures correspondant au détail repéré VII des figures respectives 4a à 5c.

### DESCRIPTION DÉTAILLÉE D'UN MODE DE RÉALISATION PRÉFÉRENTIEL DE L'INVENTION

En référence aux dessins, on va maintenant décrire un exemple de réalisation de l'invention.

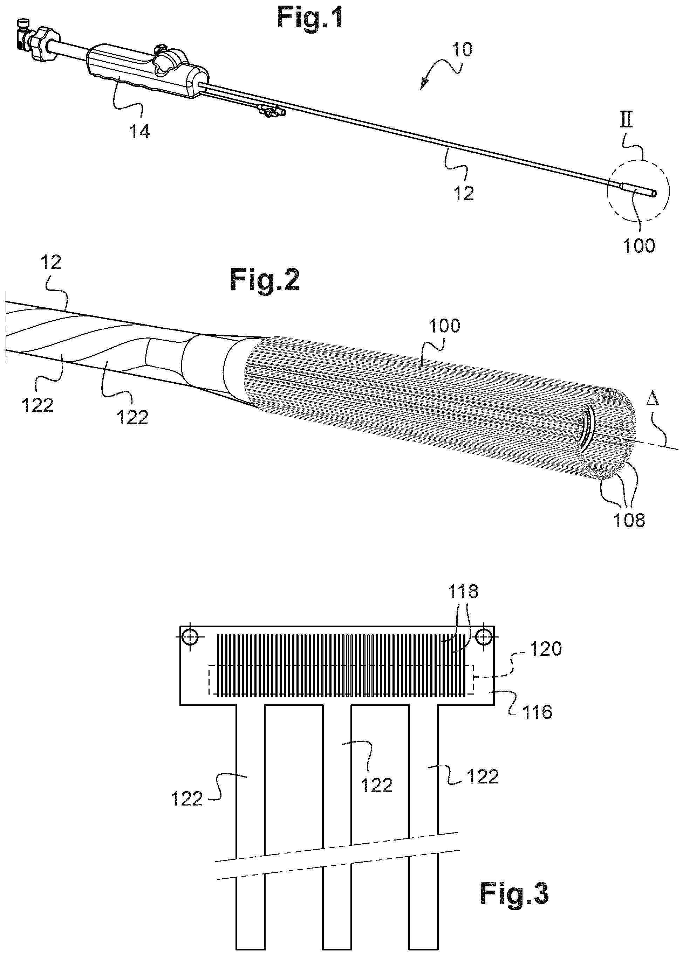

La Figure 1 est une vue d'ensemble d'un instrument d'exploration et d'intervention intracorporelles selon l'invention. L'instrument 10 comprend un cathéter téléorientable 12 relié à son extrémité proximale à une poignée de manoeuvre 14 et à son extrémité opposée, distale, à un dispositif chirurgical destiné à intervenir sur un site précisément repéré à l'intérieur du corps d'un patient, ou à explorer ce site par un moyen d'observation ou d'imagerie permettant de visualiser la région située face à l'extrémité distale du cathéter téléorientable 12.

Ici et dans la suite, le terme "proximal" (ou "arrière") sera considéré par rapport à l'instrument dans son ensemble, c'est-à-dire en direction de la poignée 14 manipulée par le praticien ; de la même façon, le terme "distal" (ou "avant") se référera à une direction opposée, donc proche du site d'intervention ou d'exploration. Sur les différentes figures annexées, ces directions proximale et distale correspondent respectivement à la gauche et à la droite. De même, le terme "axial" sera utilisé en référence à l'axe Δ du cathéter 12, c'est-à-dire la plus grande dimension de celui-ci, une direction "radiale" étant une direction située dans un plan perpendiculaire à la direction axiale Δ.

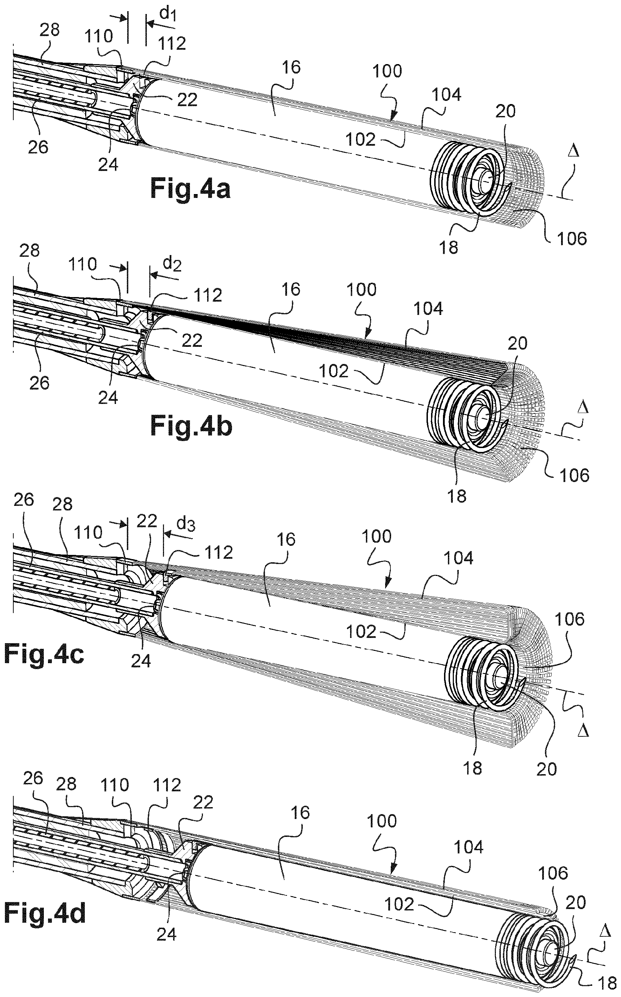

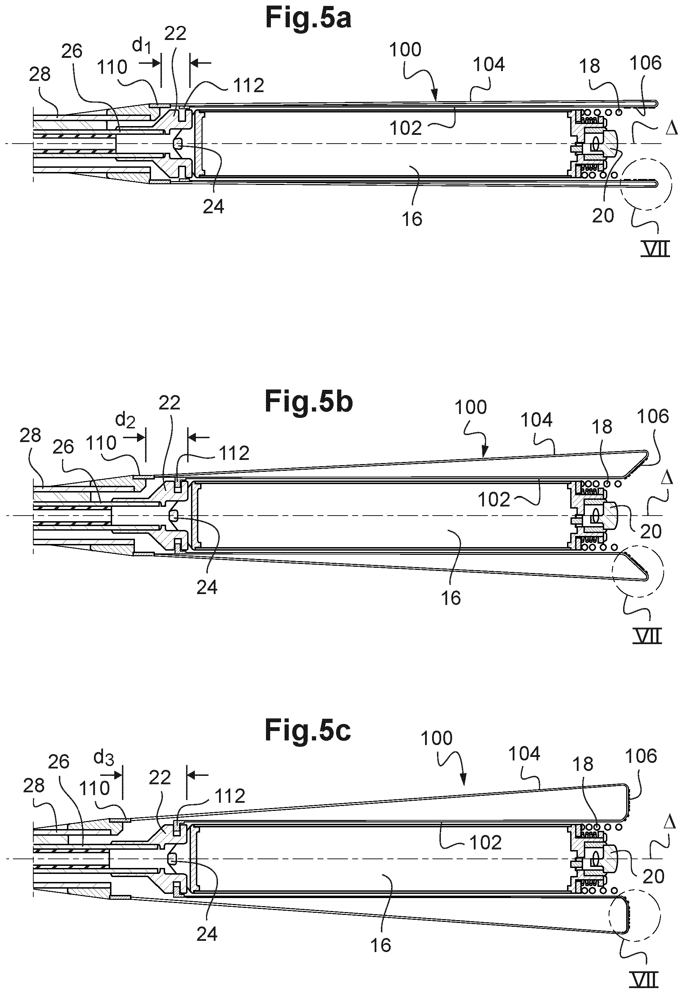

Dans une forme particulière, non imitative, de mise en oeuvre de l'invention, qui est celle illustrée dans le cadre de la présente description et des figures annexées, le cathéter téléorientable 12 est utilisé pour implanter une capsule autonome leadless 16 (Figures 4a à 4d et 5a à 5c) du type décrit en introduction de la présente description.

Il peut notamment s'agir d'une capsule à implanter dans le ventricule droit d'un coeur, au fond de ce ventricule dans la région de l'apex. Dans ce cas, l'accès au ventricule droit se fait par la veine cave via le sinus coronaire puis la valve tricuspide, suivant une procédure en elle-même bien connue et décrite par exemple dans le US 2014/0378991 (Ollivier). Le cathéter téléorientable 12 porte à cet effet à son extrémité distale un tube ou "housing" logeant et protégeant la capsule pendant la progression dans le réseau veineux et au passage de la valve, et protégeant les tissus environnants des risques de déchirements potentiels avant que la capsule n'atteigne sa position définitive face au site d'implantation où la capsule sera alors progressivement déployée hors du housing 100 jusqu'à accostage avec la paroi cardiaque.

La capsule 16 comprend de façon en elle-même connue un corps tubulaire pourvu à l'une de ses extrémités d'une vis d'ancrage hélicoïdale saillante 18 prolongeant axialement le corps tubulaire et solidaire de celui-ci en rotation. La vis d'ancrage comprend dans sa partie distale une longueur de l'ordre de 1,5 à 2 mm de spires non jointives, destinées à pénétrer dans le tissu cardiaque de façon à y assujettir la capsule et plaquer une électrode 20 contre le tissu de manière à pouvoir recueillir les signaux de dépolarisation cardiaque et appliquer éventuellement des impulsions de stimulation en cet endroit.

Du côté proximal, l'extrémité arrière 24 de la capsule est couplée à un embout 22 de cathéter formant organe d'accouplement conjugué. L'embout 22 est relié à un tube mobile interne 26 logé à l'intérieur d'un tube intermédiaire 28. Le tube mobile 26 et le tube intermédiaire 28 sont coaxiaux et s'étendent d'une extrémité proximale à une extrémité distale du cathéter téléorientable 12 en étant montés télescopiquement l'un dans l'autre avec possibilité de rotation mutuelle et de translation axiale mutuelle. Le tube intermédiaire 28 est solidaire du corps de la poignée de manoeuvre 14, tandis que le tube interne 26 est déplaçable en rotation et en translation par rapport au tube intermédiaire 28 au moyen de diverses manettes et boutons à disposition du praticien sur la poignée 14.

Une telle structure de cathéter est notamment décrite dans les demandes (ultérieurement publiées) EP 19199991.1 et US 16/237,749 et, en ce qui concerne la poignée, dans les demandes (ultérieurement publiées) EP 19202177.2 et US 16/416,201.

Essentiellement, pour implanter la capsule le praticien fait progresser le cathéter-guide 12 le long de la veine cave puis oriente l'extrémité distale de celui-ci de façon précise jusqu'à ce que l'extrémité distale portant le housing 100 accoste au fond du ventricule. Le praticien opère alors à l'aide des diverses manettes et boutons de la poignée une manoeuvre de translation de la capsule en direction distale, qui déploie cette dernière hors du housing 100. Il transmet alors à la capsule, via le tube interne 26 et l'embout d'accouplement 22, un mouvement combiné de translation, pour appuyer l'extrémité distale de la capsule contre la paroi, et de rotation, pour visser la capsule de manière à l'ancrer dans cette paroi. La rotation est poursuivie jusqu'à ce que la face avant de la capsule, qui porte l'électrode 20, vienne en appui contre la paroi ; cette position est la position définitive de la capsule. Le praticien découple alors l'embout 22 porté par le tube interne 26 d'avec la capsule, puis procède au retrait du cathéter-guide 12 hors de l'organisme par une manoeuvre inverse de celle qui avait permis l'implantation.

L'invention concerne plus précisément la configuration particulière du tube d'extrémité distale 100, qui dans l'application illustrée est le housing entourant et protégeant la capsule leadless jusqu'au site d'implantation. Ce tube d'extrémité doit répondre à la double contrainte :
d'être creux, c'est-à-dire de laisser subsister un large volume interne libre pour pouvoir y loger la capsule à implanter et permettre le coulissement du tube interne mobile 26 pendant le déploiement de la capsule vers le site d'implantation ; et
de supporter sur une face frontale, distale, un réseau de transducteurs, par exemple de transducteurs à ultrasons, avec l'axe principal du diagramme de rayonnement des transducteurs qui puissent être orientés en direction du site d'implantation, de manière à pouvoir reconstituer une image de ce site à partir de signaux délivrés par les transducteurs du réseau et transmis à un dispositif externe d'imagerie pour traitement par un logiciel approprié et affichage sur un écran à la disposition du praticien.

L'invention propose à cet effet de réaliser le tube 100 sous la forme d'un tube déformable à la manière d'un doigt de gant. Plus précisément, le tube déformable 100 est un tube à double paroi, avec :
un manchon interne 102 (Figures 4a à 4d et 5a à 5c), s'étendant d'une extrémité proximale à une extrémité distale et définissant une lumière axiale, en l'espèce un volume de housing recevant la capsule leadless 16, débouchant côté proximal (vers l'embout d'accouplement 22 dans l'exemple illustré) et côté distal (vers le site d'implantation visé) ;
un manchon externe 104, coaxial au manchon interne et s'étendant d'une extrémité proximale à une extrémité distale ; et
un module d'imagerie 106, attenant à la fois au manchon interne 102 et au manchon externe 104, ce module d'imagerie 106 étant de forme annulaire, relié en périphérie interne à l'extrémité distale du manchon interne et en périphérie externe à l'extrémité distale du manchon externe.

Très avantageusement, dans une forme de réalisation particulière illustrée notamment sur la Figure 2 et les Figures 4a à 4d, le tube déformable 100 est constitué d'un ensemble de lamelles flexibles parallèles s'étendant chacune dans un plan axial, suivant des génératrices du tube déformable 100. Chacune des lamelles flexibles 108 est formée d'une seule pièce de matériau élastique, par exemple d'un métal ou alliage métallique, avantageusement de titane. Chacune des lamelles 108 s'étend de l'extrémité proximale du manchon interne 102 jusqu'à l'extrémité proximale du manchon externe 104 en étant repliée sur elle-même au niveau du module d'imagerie 106. Les extrémités proximales de la partie des lamelles 108 formant le manchon externe 104 sont réunies ensemble à une bague d'extrémité externe 110, fixée à l'extrémité distale du tube intermédiaire 28, tandis que les extrémités proximales de la partie des lamelles 108 formant le manchon interne 102 sont réunies ensemble à une bague d'extrémité 112 couplée, mais non solidarisée, à l'embout 22, donc au tube interne 26.

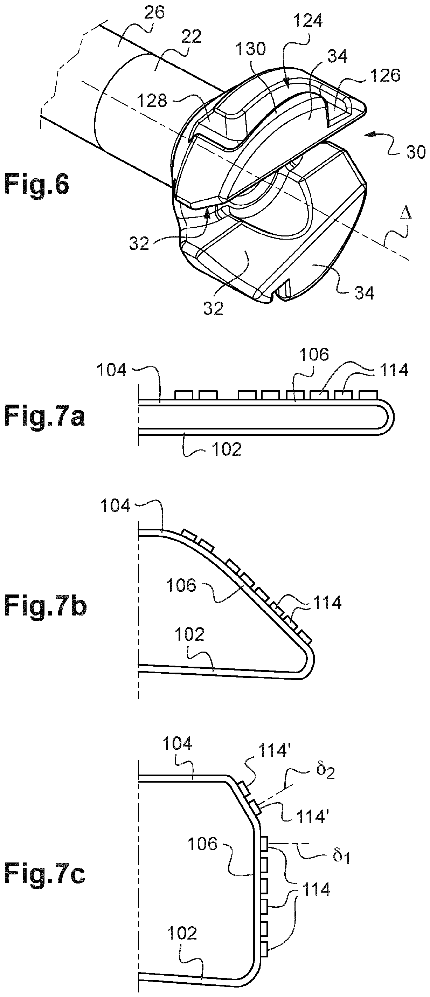

Le module d'imagerie 106 porte le réseau de transducteurs 114 (Figures 7a à 7c). Ces transducteurs sont avantageusement montés sur un circuit imprimé flexible 116 portant et interconnectant les transducteurs du réseau.

Un transducteurs à ultrasons comprenant des puces reportées sur un circuit flexible est décrit par exemple dans le US 2004/0054289 A1, auquel on pourra se reporter pour de plus amples détails sur les aspects technologiques de la réalisation. Dans le cas particulier de l'invention, le circuit imprimé flexible 116 porte comme illustré Figure 3 une série de pistes conductrices 118 sur lesquelles sont reportés ou détourés les éléments de transducteur, par exemple des puces CMUT (*Capacitive Micromachined Ultrasonic Transducer*) ou piézoélectriques. Les transducteurs sont montés dans une zone 120 du circuit flexible ; le circuit porte par exemple huit lignes de 64 colonnes formant un réseau matriciel de 512 transducteurs, par exemple de transducteurs à ultrasons réalisés sous forme de puces élémentaires de 0,25 x 0,25 mm. Le circuit imprimé flexible 116 est prolongé côté proximal par un ou plusieurs rubans 122 portant des pistes conductrices de liaison aux transducteurs du réseau. La configuration de ces rubans permet, comme cela est visible Figure 2, de les enrouler côte à côte à la surface du cathéter téléorientable 12 d'une extrémité à l'autre de celui-ci, ce qui permet de relier électriquement les transducteurs du réseau situés à l'extrémité distale à un connecteur électrique situé à l'extrémité opposée, proximale, du cathéter 12 au niveau de la poignée de manoeuvre 14. Le circuit imprimé flexible 116 peut être un circuit imprimé multicouche pour augmenter le nombre de conducteurs de liaison portés par chaque ruban 122. Avantageusement, l'épaisseur du circuit est inférieure à 20 µm, pour ne pas entraver la déformation du tube déformable 100 notamment au niveau du module d'imagerie 106 et permettre un enroulement aisé des rubans 122 à la surface du cathéter téléorientable 12. En outre, des parties rigides peuvent être accrochées sur tout ou partie des rubans, tant sur la face ou se trouvent les transducteurs qu'au dos du dispositif, pour favoriser un pliage reproductible et contrôlé. Des éléments bistables ou à mémoire de forme peuvent être également installés sous la partie active, pour former une courbure contrôlable assurant un contrôle géométrique de la focalisation du réseau d'éléments.

On va maintenant décrire la cinématique de déformation du tube à double paroi 100, en référence notamment aux Figures 4a à 4d (vues en perspective partiellement arrachées), 5a à 5c (correspondant aux Figures 4a à 4c, en coupe par un plan axial) et 7a à 7c (correspondant au détail repéré VII sur les figures respectives 5a à 5c).

La position initiale, dite position repliée, est celle illustrée sur les Figures 4a, 5a et 7a.

Dans cette position, le manchon interne 102 et le manchon externe 104 présentent tous deux une forme cylindrique, et le module d'imagerie 106 s'étend substantiellement dans le prolongement du manchon interne 102. Plus précisément, côté proximal, la bague interne 112 est rapprochée de la bague externe 110 d'une distance d₁ et, côté distal, les transducteurs 114 (cf. Figure 7a) supportés par le module d'imagerie 106 sont tournés vers l'intérieur du tube 100, donc protégés vis-à-vis de l'extérieur. On notera, dans cette position repliée, l'existence d'une marge de sécurité importante dans la distance entre l'extrémité de la vis 18 de la capsule leadless et l'extrémité du tube déformable 100 constituant le housing de protection de cette capsule ; les tissus corporels environnants sont ainsi parfaitement protégés lors de la progression du tube, par exemple dans le réseau veineux.

Les Figures 4b, 5b et 7b illustrent une position intermédiaire.

Dans cette position, le tube interne mobile 26 est progressivement repoussé vers l'avant à l'intérieur du tube intermédiaire 28 par une manoeuvre du praticien depuis la poignée 14 située hors du corps du patient, à l'extrémité du cathéter téléorientable 18. Cette manoeuvre a pour effet d'éloigner la bague interne 112 de la bague externe 110, ces deux bagues étant alors écartées d'une distance axiale d₂, supérieure à d₁.

Sous l'effort axial exercé par le tube mobile 26 repoussé par le praticien faisant plier les lamelles flexibles 108 du tube déformable 100, la structure va progressivement se développer en raison de la longueur développée constante des trois éléments attenants constitués par (i) le manchon interne 102, (ii) le module d'imagerie 106 et (iii) le manchon externe 104 (c'est-à-dire, concrètement, de la longueur totale de chacune des lamelles flexibles 108), ainsi que par effet de charnière à la jonction (i) des éléments 102 et 106, et (ii) des éléments 106 et 104. Le manchon interne 102 garde sa forme cylindrique mais, du fait de la longueur développée constante précitée, le manchon externe 104 se déformer radialement vers l'extérieur dans sa partie la plus distale, en prenant une forme progressivement conique, d'axe Δ. Il en résulte un redressement du module 106 qui se déplie en corolle, en prenant également une forme conique d'axe Δ.

On notera que cette forme de corolle peut être rendue torique par adjonction de couches supplémentaires de gestion de la contrainte du module permettant de modifier de manière contrôlée le profil de déformation de celui-ci, en fonction d'une loi de focalisation géométrique souhaitée pour le réseau de transducteurs, allant de la focalisation à la défocalisation pour création d'un faisceau divergent.

La position finale, dite position déployée, est celle illustrée sur les Figures 4c, 5c et 7c.

Dans cette position, le manchon interne 102 présente toujours une forme cylindrique, le manchon externe 104 présente une forme conique, plus évasée que dans la position intermédiaire de la Figure 5b, et le module d'imagerie 106 s'étend maintenant substantiellement avec la face portant les transducteurs 114 (cf. notamment Figure 7c) tournée en direction axiale vers l'avant. Les bagues interne 112 et externe 110 sont écartées d'une distance d₃, supérieure à d₂. Typiquement, la course complète d₃ - d₁ de la bague interne 112 par rapport à la bague externe 110 est de l'ordre de quelques millimètres, par exemple d₃ - d₁ = 3,1 mm.

Dans cette position déployée les transducteurs 114 ont l'axe principal δ₁ de leur diagramme de rayonnement orienté parallèlement à l'axe Δ, ce qui correspond sur le plan fonctionnel à une configuration du réseau de transducteurs de type *front looking.* Cette configuration permet d'émettre et de recevoir des signaux qui, après un traitement d'imagerie approprié, fourniront une représentation visualisable du site d'implantation face à l'extrémité du tube déformable 100, ce dernier jouant toujours, dans cette position, le rôle d'un housing qui loge et protège le corps de la capsule leadless 16 à implanter.

Avantageusement, comme illustré plus précisément sur la Figure 7c, une partie des transducteurs 114, référencés 114', qui sont situés près de l'interface avec le manchon externe 104, ont l'axe principal δ₂ de leur diagramme de rayonnement qui est orienté suivant une direction inclinée par rapport à l'axe central Δ du tube déformable 100, avec par exemple une inclinaison de l'ordre de 30°. Ceci permet de recueillir des signaux d'imagerie correspondant à des points sur l'axe Δ en avant de l'extrémité du tube déformable 100, à proximité du site d'implantation visé.

On notera, dans cette position déployée, la faible distance entre l'extrémité de la vis 18 de la capsule leadless et le site d'implantation où cette vis devra être ancrée.

Une fois les signaux d'imagerie recueillis et analysés, si le praticien considère que le site d'implantation visé est un site satisfaisant, il procède alors à l'extraction de la capsule leadless 16 hors de son housing (constitué par le tube déformable 100 dans la configuration des Figures 4c et 5c), et repousse l'embout 22 en direction distale par poursuite du mouvement de translation axiale du tube interne 26 dans le tube intermédiaire 28.

Une fois l'extrémité de la vis d'ancrage 18 venue en contact avec la paroi cardiaque, le praticien imprime au tube interne mobile 26 un mouvement de rotation pour ancrer en place la capsule par vissage dans les tissus de cette paroi.

Avantageusement, le système est agencé de manière que la poursuite de ce mouvement de rotation réalise automatiquement un découplage de l'embout 22 d'avec la bague interne 112 à laquelle il était resté couplé pendant la manoeuvre décrite plus haut (couplage qui, précisément, permettait le déploiement face au site d'implantation du module d'imagerie 106 du tube déformable 100, par translation du tube interne mobile 26).

Pour ce faire, l'embout 22 présente une structure telle que celle illustrée Figure 6, qui est dérivée d'une forme d'embout décrite dans les demandes (ultérieurement publiées) EP 19202358.8 et US 16/655,198, avec une empreinte recevant l'extrémité arrière 24 de la capsule 16 et constituée d'un évidement 30 symétrique en forme approximative de V, avec deux faces inclinées 32 s'étendant selon des plans situés de part et d'autre de l'axe central Δ en une configuration de dièdre symétrique, l'axe de ce dièdre s'étendant dans une direction radiale. L'embout 22 comprend en outre une surface frontale tournée vers la capsule, qui est constituée de deux demi-faces frontales 34 qui viennent prolonger en direction radiale chaque face inclinée respective 32 de l'évidement 30. Les demi-faces frontales 34 sont inclinées dans des sens opposés par rapport à un plan radial, d'un angle compris entre 5 et 15°, dans un sens pour l'une des demi-faces frontales 34 et dans le sens opposé pour l'autre demi-face frontale 34.

Selon la présente invention, l'embout 22 comprend en outre deux cames périphériques 124 diagonalement opposées, destinées à recevoir deux dents diamétralement opposées homologues de la bague interne 112.

Chacune de ces cames 124 présente une forme en S, avec (i) une première portion 126 ouverte vers l'avant et (ii) une seconde portion 128 ouverte vers l'arrière, ces deux portions étant angulairement décalées, orientées parallèlement à l'axe Δ, et raccordées par (iii) une troisième portion 130 s'étendant selon une direction circonférentielle. Le mouvement de rotation imprimé à l'embout 22 au moment du vissage aura ainsi pour effet de faire passer le doigt de la bague interne 112 de la première portion 126 à la seconde portion 128. de la came et désengager ainsi la bague interne 112 de l'embout 22.

Du fait de l'élasticité des lamelles flexibles 108, le tube déformable 110 reprendra alors sa forme initiale (correspondant à la position repliée), comme illustré Figure 4d. L'extrémité arrière 24 de la capsule 16 reste en revanche couplée à l'embout 22, ce qui permet la poursuite du vissage de la capsule (procédure de pose standard) avec en fin de course limitation du couple de vissage et découplage de la capsule d'avec l'embout 22, de la manière décrite dans les documents EP 19202358.8 et US 16/655,198 précités.

Des dimensions typiques du tube déformable 100, pour une application à l'implantation d'une capsule leadless, sont, dans la configuration illustrée Figure 2 (position repliée) :
diamètre hors-tout : 25 French (8,33 mm) pour l'implantation d'une capsule de diamètre 21 French (7 mm) ;
longueur : 30 à 50 mm,
Le manchon étant formé de 64 lamelles minces 108 en titane, sur
lesquelles est collé le circuit imprimé flexible 116.

On notera que, dans l'exemple décrit plus haut, la configuration de déploiement est celle d'un manchon interne 102 qui reste cylindrique, tandis que le manchon externe 104, initialement cylindrique, prend une forme progressivement conique au cours du déploiement.

En variante, il est possible d'envisager la configuration inverse, c'est-à-dire une configuration où le manchon externe 104 garde sa forme cylindrique initiale et où c'est le manchon interne 102 qui se déploie avec une forme progressivement conique, le cône étant orienté avec son plus petit diamètre côté distal. Cette variante est envisageable dans une application autre que l'implantation d'une capsule leadless, par exemple pour une intervention chirurgicale où l'instrument d'intervention n'est introduit dans le cathéter téléorientable 12 qu'après exécution des étapes de déploiement du réseau de transducteurs ; dans ce cas, le manchon interne 102 n'a pas besoin de jouer un rôle de housing, et il suffit qu'en position déployée le diamètre de la lumière centrale soit suffisant pour permettre le passage de l'instrument chirurgical.

## Revendications

1. Un instrument (10) d'exploration et d'intervention intracorporelles, comprenant :
un tube déformable (100) à double paroi, comprenant :
un manchon interne (102) s'étendant d'une extrémité proximale à une extrémité distale, et définissant une lumière axiale débouchant côté distal et côté proximal ;
un manchon externe (104), coaxial au manchon interne (102) et s'étendant d'une extrémité proximale à une extrémité distale ; et
un module d'imagerie (106), relié en périphérie interne à l'extrémité distale du manchon interne (102) et en périphérie externe à l'extrémité distale du manchon externe (104) ; et
un réseau de transducteurs à ultrasons (114), portés par une face support externe du module d'imagerie (106);
et
le module d'imagerie (106) est un module annulaire relié avec effet de charnière à sa jonction avec i) l'extrémité distale du manchon interne (102) et avec ii) l'extrémité distale du manchon externe (104),
le manchon interne (102) et le manchon externe (104) sont mobiles en translation axiale relative sous l'effet d'un effort axial, et
le manchon interne (102), le manchon externe (104) et le module annulaire d'imagerie (106) sont attenants et présentent une longueur développée constante;
et
dans une position repliée, le manchon interne (102) et le manchon externe (104) présentent tous deux une forme cylindrique, et le module annulaire d'imagerie (106) s'étend substantiellement dans le prolongement de l'un du manchon interne (102) ou du manchon externe (104) ; et
dans une position déployée, l'un du manchon interne (102) présente une forme cylindrique et l'autre du manchon interne (102) ou du manchon externe (104) présente une forme évasée s'élargissant côté distal, et le module annulaire d'imagerie (106) s'étend substantiellement avec la face portant les transducteurs du réseau tournée en direction axiale vers l'avant du tube déformable.

2. L'instrument de la revendication 1, dans lequel les transducteurs (114) sont montés sur le module annulaire d'imagerie (106) avec l'axe de leur diagramme de rayonnement orienté perpendiculairement à la face support.

3. L'instrument de la revendication 1,
dans lequel en position repliée les extrémités proximales respectives du manchon interne (102) et du manchon externe (104) sont mutuellement rapprochées, et le module annulaire d'imagerie (106) s'étend substantiellement dans le prolongement du manchon interne (102), au moins une partie de la face du module annulaire d'imagerie (106) portant les transducteurs du réseau étant orientée radialement vers l'intérieur du tube déformable (100),
et dans lequel en position déployée les extrémités proximales respectives du manchon interne (102) et du manchon externe (104) sont mutuellement éloignées, la face du module annulaire d'imagerie (106) portant les transducteurs du réseau s'étendant au moins en partie dans un plan radial où les transducteurs sont orientés suivant une direction axiale parallèle à l'axe central du tube déformable.

4. L'instrument de la revendication 3, dans lequel en position déployée la face du module annulaire d'imagerie (106) portant les transducteurs (114) du réseau s'étend avec une partie des transducteurs (114) orientés suivant une direction axiale parallèle à l'axe central du tube déformable, et une autre partie des transducteurs orientés suivant une direction inclinée intersectant l'axe central du tube déformable.

5. L'instrument de la revendication 1, dans lequel le module annulaire d'imagerie (106) comprend un circuit imprimé flexible (116) portant et interconnectant les transducteurs (114) du réseau.

6. L'instrument de la revendication 5, dans lequel le circuit imprimé flexible (116) est prolongé côté proximal par un ou plusieurs rubans (122) s'étendant jusqu'à une extrémité proximale du tube déformable et au-delà, les rubans portant des pistes conductrices de liaison aux transducteurs (114) du réseau.

7. L'instrument de la revendication 1, dans lequel le tube déformable comprend un ensemble de lamelles flexibles (108) parallèles s'étendant chacune dans un plan axial,
dans lequel chacune des lamelles (108) est formée d'une seule pièce en matériau élastique,
et dans lequel chacune des lamelles (108) s'étend de l'extrémité proximale du manchon interne (102) jusqu'à l'extrémité proximale du manchon externe (104) en étant repliée sur elle-même au niveau du module annulaire d'imagerie (106).

8. L'instrument de la revendication 7, dans lequel le matériau élastique des lamelles (108) est un métal ou un alliage métallique.

9. L'instrument de la revendication 1, dans lequel le manchon interne (102) et le manchon externe (104) comportent chacun une bague (110, 112) à leur extrémité proximale respective, un déplacement des deux bagues en translation axiale relative réalisant une transition entre la position repliée et la position déployée.

10. L'instrument de la revendication 1, comprenant en outre un cathéter téléorientable (12) prolongeant le tube déformable à son extrémité proximale, le cathéter téléorientable comprenant un tube mobile (26) logé à l'intérieur d'un tube intermédiaire (28),
le tube mobile (26) et le tube intermédiaire (28) étant coaxiaux, s'étendant d'une extrémité proximale à une extrémité distale du cathéter et étant montés télescopiquement l'un dans l'autre avec possibilité de rotation mutuelle et de translation axiale mutuelle,
et dans lequel le manchon interne (102) et le manchon externe (104) sont reliés en partie proximale respectivement au tube mobile (26) et au tube intermédiaire (28) du cathéter.

11. L'instrument de la revendication 10, dans lequel le manchon interne (102) est relié de façon découplable au tube mobile (26) du cathéter.

12. L'instrument de la revendication 11, dans lequel le manchon interne (102) est relié au tube mobile (26) du cathéter par l'intermédiaire d'un embout (22) comportant un organe d'accouplement du tube mobile du cathéter à une capsule autonome implantable (16) apte à être logée à l'intérieur de la lumière axiale du manchon interne (102).

13. L'instrument de la revendication 12, dans lequel l'embout (22) est découplable du manchon interne (102) sous l'effet d'une rotation du tube mobile (26) dans le tube intermédiaire (28) du cathéter, l'embout restant couplé au manchon interne (102) pendant une translation axiale du tube mobile (26) dans le tube intermédiaire (28) du cathéter.

14. L'instrument de la revendication 10, dans lequel le tube mobile du cathéter est relié à un instrument chirurgical apte à être logé à l'intérieur de la lumière axiale du manchon interne.

## Patentansprüche

1. Instrument (10) für intrakorporale Exploration und Intervention, umfassend:
ein doppelwandiges verformbares Rohr (100), umfassend:
eine innere Hülse (102), die sich von einem proximalen Ende zu einem distalen Ende hin erstreckt und einen axialen Schlitz definiert, der distalseitig und proximalseitig ausmündet;
eine äußere Hülse (104), die zu der inneren Hülse (102) koaxial ist und sich von einem proximalen Ende zu einem distalen Ende hin erstreckt; und
ein Bildgebungsmodul (106), das am inneren Umfang mit dem distalen Ende der inneren Hülse (102) und am äußeren Umfang mit dem distalen Ende der äußeren Hülse (104) verbunden ist; und
ein Array aus Ultraschall-Wandlern (114), die von einer äußeren Trägerfläche des Bildgebungsmoduls (106) getragen sind; und
das Bildgebungsmodul (106) ein ringförmiges Modul ist, das mit Scharnierwirkung an seiner Verbindungsstelle mit i) dem distalen Ende der inneren Hülse (102) und mit ii) dem distalen Ende der äußeren Hülse (104) verbunden ist,
die innere Hülse (102) und die äußere Hülse (104) unter der Einwirkung einer axialen Kraft in axialer Translation beweglich sind, und
die innere Hülse (102), die äußere Hülse (104) und das ringförmige Bildgebungsmodul (106) angrenzend sind und eine konstante entwickelte Länge aufweisen; und
in einer eingezogenen Stellung, die innere Hülse (102) und die äußere Hülse (104) beide eine zylindrische Form aufweisen und das ringförmige Bildgebungsmodul (106) sich im Wesentlichen in der Verlängerung einer von der inneren Hülse (102) oder der äußeren Hülse (104) erstreckt; und
in einer ausgezogenen Stellung, die eine der inneren Hülse (102) eine zylindrische Form aufweist und die andere der inneren Hülse (102) oder der äußeren Hülse (104) eine aufgeweitete Form aufweist, die sich distalseitig erweitert, und das ringförmige Bildgebungsmodul (106) sich im Wesentlichen mit der Fläche, die die Wandler des Arrays trägt, in axialer Richtung gerichtet zu dem Vorderteil des verformbaren Rohrs hin erstreckt.

2. Instrument nach Anspruch 1, wobei die Wandler (114) auf dem ringförmigen Bildgebungsmodul (106) mit der Achse ihres Strahlungsdiagramms senkrecht zu der Trägerfläche orientiert gelagert sind.

3. Instrument nach Anspruch 1, wobei in eingezogener Stellung die jeweiligen proximalen Enden der inneren Hülse (102) und der äußeren Hülse (104) gegenseitig angenähert sind und das ringförmige Bildgebungsmodul (106) sich im Wesentlichen in der Verlängerung der inneren Hülse (102) erstreckt, wobei wenigstens ein Teil der Fläche des ringförmigen Bildgebungsmoduls (106), die die Wandler des Arrays trägt, radial zu dem Inneren des verformbaren Rohrs (100) hin orientiert ist,
und wobei in ausgezogener Stellung die jeweiligen proximalen Enden der inneren Hülse (102) und der äußeren Hülse (104) gegenseitig beabstandet sind, wobei die Fläche des ringförmigen Bildgebungsmoduls (106), die die Wandler des Arrays trägt, sich wenigstens zum Teil in einer radialen Ebene erstreckt, wo die Wandler gemäß einer axialen zu der mittigen Achse des verformbaren Rohrs parallelen Richtung orientiert sind.

4. Instrument nach Anspruch 3, wobei in ausgezogener Stellung die Fläche des ringförmigen Bildgebungsmoduls (106), die die Wandler (114) des Arrays trägt, sich mit einem Teil der Wandler (114), die gemäß einer axialen zu der mittigen Achse des verformbaren Rohrs parallelen Richtung orientiert sind, und einem anderen Teil der Wandler, die gemäß einer geneigten Richtung, welche die mittige Achse des verformbaren Rohrs schneidet, erstreckt.

5. Instrument nach Anspruch 1, wobei das ringförmige Bildgebungsmodul (106) eine flexible Leiterplatte (116) umfasst, die die Wandler (114) des Arrays trägt und vernetzt.

6. Instrument nach Anspruch 5, wobei die flexible Leiterplatte (116) sich proximalseitig über ein oder mehrere Bänder (122) fortsetzt, die sich bis zu einem proximalen Ende des verformbaren Rohrs und darüber hinaus erstrecken, wobei die Bänder Leiterbahnen für die Verbindung mit den Wandlern (114) des Arrays tragen.

7. Instrument nach Anspruch 1, wobei das verformbare Rohr eine Anordnung aus parallelen flexiblen Lamellen (108) umfasst, die sich jede in einer axialen Ebene erstrecken,
wobei jede der Lamellen (108) aus einem Stück aus elastischem Material gebildet ist,
und wobei jede der Lamellen (108) sich von dem proximalen Ende der innere Hülse (102) zu dem proximalen Ende der äußeren Hülse (104) hin erstreckt, indem sie im Bereich des ringförmigen Bildgebungsmoduls (106) auf sich selbst umgeschlagen ist.

8. Instrument nach Anspruch 7, wobei das elastische Material der Lamellen (108) ein Metall oder eine metallische Legierung ist.

9. Instrument nach Anspruch 1, wobei die innere Hülse (102) und die äußere Hülse (104) jede einen Ring (110, 112) an ihrem jeweiligen proximalen Ende tragen, wobei eine Bewegung der zwei Ringe in relativer axialer Translation einen Übergang zwischen der umgeschlagenen Stellung und der ausgezogenen Stellung realisiert.

10. Instrument nach Anspruch 1, ferner umfassend einen fernsteuerbaren Katheter (12), der das verformbare Rohr an seinem proximalen Ende fortsetzt, wobei der fernsteuerbare Katheter ein bewegliches Rohr (26) umfasst, das im Inneren eines Zwischenrohres (28) aufgenommen ist,
wobei das bewegliche Rohr (26) und das Zwischenrohr (28) koaxial sind, sich von einem proximalen Ende zu einem distalen Ende des Katheters hin erstrecken und teleskopisch das eine in dem anderen mit der Möglichkeit der gegenseitigen Rotation und der gegenseitigen axialen Translation gelagert sind
und wobei die innere Hülse (102) und die äußere Hülse (104) im proximalen Teil mit dem beweglichen Rohr (26) bzw. mit dem Zwischenrohr (28) des Katheters verbunden sind.

11. Instrument nach Anspruch 10, wobei die innere Hülse (102) entkoppelbar mit dem beweglichen Rohr (26) des Katheters verbunden ist.

12. Instrument nach Anspruch 11, wobei die innere Hülse (102) mit dem beweglichen Rohr (26) des Katheters mittels eines Ansatzstücks (22) verbunden ist, das ein Organ zum Ankoppeln des beweglichen Rohrs des Katheters an eine implantierbare autonome Kapsel (16) aufweist, die im Inneren des axialen Schlitzes der inneren Hülse (102) aufnehmbar ist.

13. Instrument nach Anspruch 12, wobei das Ansatzstück (22) von der inneren Hülse (102) unter der Einwirkung einer Rotation des beweglichen Rohrs (26) in dem Zwischenrohr (28) des Katheters entkoppelbar ist, wobei das mit der inneren Hülse (102) gekoppelte Ansatzstück während einer axialen Translation des beweglichen Rohrs (26) in dem Zwischenrohr (28) des Katheters gekoppelt bleibt.

14. Instrument nach Anspruch 10, wobei das bewegliche Rohr des Katheters mit einem chirurgischen Instrument verbunden ist, das im Inneren des axialen Schlitzes der inneren Hülse aufnehmbar ist.

## Claims

1. An instrument (10) for intracorporeal exploration and intervention, comprising:
a dual-wall deformable tube (100), comprising:
an inner sleeve (102) extending from a proximal end to a distal end, and defining an axial lumen which opens on a distal side and on a proximal side;
an outer sleeve (104), coaxial to the inner sleeve (102) and extending from a proximal end to a distal end; and
an imaging module (106), connected at an inner periphery to the distal end of the inner sleeve (102) and at an outer periphery to the distal end of the outer sleeve (104); and
an array of ultrasound transducers (114), carried by an outer support face of the imaging module (106); and
the imaging module (106) is an annular module connected with an hinge effect at its junction with (i) the distal end of the inner sleeve (102) and with (ii) the distal end of the outer sleeve (104),
the inner sleeve (102) and the outer sleeve (104) are mobile in relative axial translation under the effect of an axial effort, and
the inner sleeve (102) and the outer sleeve (104) are adjacent and have a constant developed length; and
in a folded position, both the inner sleeve (102) and the outer sleeve (104) have a cylindrical shape, and the imaging module (106) substantially extends in the continuation of one of the inner sleeve (102) or the outer sleeve (104); and
in an expanded position, one of the inner sleeve (102) has a cylindrical shape and the other of the inner sleeve (102) or the outer sleeve (104) has a flared shape widening on the distal side, and the imaging module (106) substantially extends with the support face that carries the transducers of the array turned in the axial direction towards the front of the deformable tube.

2. The instrument of claim 1, wherein the transducers (114) are mounted on the imaging module (106) with the axis of their radiation diagram directed perpendicular to the support face.

3. The instrument of claim 1, wherein in the folded position the respective proximal ends of the inner sleeve (102) and of the outer sleeve (104) are moved closer to each other, and the imaging module (106) substantially extends in the continuation of the inner sleeve (102), at least a part of the support face of the imaging module (106) that carries the transducers of the array being directed radially towards an inside of the deformable tube (100);
and wherein in the expanded position, the respective proximal ends of the inner sleeve (102) and of the outer sleeve (104) are spaced apart from each other, the support face of the imaging module (106) that carries the transducers of the array extending at least partly in a radial plane in which the transducers are directed in the axial direction parallel to a central axis of the deformable tube.

4. The instrument of claim 3, wherein in the expanded position the support face of the imaging module (106) that carries the transducers (114) of the array extends with a part of the transducers (114) being directed in an axial direction parallel to the central axis of the deformable tube, and another part of the transducers being directed in an inclined direction intersecting the central axis of the deformable tube.

5. The instrument of claim 1, wherein the imaging module (106) comprises a flexible printed circuit (116) carrying and interconnecting the transducers (114) of the array.

6. The instrument of claim 5, wherein the flexible printed circuit (116) is extended on the proximal side by one or several ribbons (122) extending up to a proximal end of the deformable tube and beyond, the at least one ribbon carrying conductive tracks for a connection to the transducers (114) of the array.

7. The instrument of claim 1, wherein the deformable tube comprises a set of parallel flexible lamellas (108) each extending in an axial plane,
wherein each of the lamellas (108) is formed of a single part made of an elastic material,
and wherein each of the lamellas (108) extends from the proximal end of the inner sleeve (102) up to the proximal end of the outer sleeve (104) by being folded over itself at the imaging module (106).

8. The instrument of claim 7, wherein the elastic material of the lamellas (108) is a metal or a metallic alloy.

9. The instrument of claim 1, wherein the inner sleeve (102) and the outer sleeve (104) each include a ring (110, 112) at their respective proximal end, a displacement of the two rings in relative axial translation enabling a transition between the folded position and the expanded position.

10. The instrument of claim 1, further comprising a steerable catheter (12) extending the deformable tube at its proximal end, the steerable catheter comprising a mobile tube (26) housed within an intermediate tube (28),
the mobile tube(26) and the intermediate tube (28) being coaxial to each other, extending from a proximal end to a distal end of the catheter and being mounted telescopically into each other with a possibility of mutual rotation and mutual axial translation,
and wherein the inner sleeve (102) and the outer sleeve (104) are respectively connected in a proximal part to the mobile tube (26) and the intermediate tube (28) of the catheter.

11. The instrument of claim 10, wherein the inner sleeve (102) is detachably connected to the mobile tube (26) of the catheter.

12. The instrument of claim 11, wherein the inner sleeve (102) is connected to the mobile tube (26) of the catheter by means of a tip (22) including a member for coupling the mobile tube of the catheter to an implantable autonomous capsule (16) adapted to be housed within the axial lumen of the inner sleeve (102).

13. The instrument of claim 12, wherein the tip (22) is adapted to be uncoupled from the inner sleeve (102) under the effect of a rotation of the mobile tube (26) in the intermediate tube (28) of the catheter, the tip remaining coupled to the inner sleeve (102) during an axial translation of the mobile tube (26) in the intermediate tube (28) of the catheter.

14. The instrument of claim 10, wherein the mobile tube of the catheter is connected to a surgical instrument adapted to be housed within the axial lumen of the inner sleeve.
